(19) Europäisches Patentamt
European Patent Office
Office européen des brevets

(11) **EP 2 201 935 B1**

## (12) EUROPEAN PATENT SPECIFICATION

(45) Date of publication and mention
of the grant of the patent:
**08.07.2020   Bulletin 2020/28**

(51) Int Cl.:
*A61K 9/107* $^{(2006.01)}$         *A61K 9/51* $^{(2006.01)}$

(21) Application number: **09290356.6**

(22) Date of filing: **13.05.2009**

(54) **Polymeric micelle composition containing a poorly soluble drug and preparation method of the same**

Polymere Mizellenzusammensetzung, die ein schlecht lösliches Arzneimittel enthält, und zugehöriges Herstellungsverfahren

Composition de micelles polymères contenant un médicament faiblement soluble et son procédé de préparation

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority:  **26.12.2008   KR 20080134506**

(43) Date of publication of application:
**30.06.2010   Bulletin 2010/26**

(73) Proprietor: **Samyang Biopharmaceuticals Corporation
Seoul 110-470 (KR)**

(72) Inventors:
- **Seo, Min-Hyo
  Daejeon-city, 302-782 (KR)**
- **Lee, Sa-Won
  Daejeon-city, 305-770 (KR)**

(74) Representative: **Novagraaf Technologies
Bâtiment O2
2, rue Sarah Bernhardt
CS90017
92665 Asnières-sur-Seine Cedex (FR)**

(56) References cited:
**WO-A1-01/85216        WO-A1-01/87345
WO-A1-02/45689        WO-A1-03/033592
WO-A1-2009/084801    WO-A1-2010/074380
US-A1- 2004 005 351    US-A1- 2007 003 625**

- **TORCHILIN V P: "Structure and design of polymeric surfactant-based drug delivery systems" JOURNAL OF CONTROLLED RELEASE, ELSEVIER, AMSTERDAM, NL LNKD-DOI:10.1016/S0168-3659(01)00299-1, vol. 73, no. 2-3, 15 June 2001 (2001-06-15), pages 137-172, XP004246457 ISSN: 0168-3659**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND**

**1. Field**

This disclosure relates to a drug-containing polymeric micelle composition and a process for preparing the same.

**2. Description of the Related Art**

Submicron particulate drug delivery systems using biodegradable polymers have been studied for the purpose of carrying out intravenous administration of drugs. Recently, it has been reported that nanoparticle systems and polymeric micelle systems using biodegradable polymers are useful technological systems that modify the *in vivo* distribution of an intravenously administrated drug to reduce undesired side effects and provide improved efficiency. Additionally, because such systems enable targeted drug delivery, they achieve controlled drug release to target organ, tissues or cells. In fact, such systems are known to have excellent compatibility with body fluids and to improve the solubilization ability of a poorly soluble drug and the drug's bioavailability.

Recently, there has been reported a process for preparing block copolymer micelles by bonding a drug chemically to a block copolymer containing a hydrophilic segment and a hydrophobic segment. The block copolymer is an A-B type diblock copolymer polymerized from a hydrophilic segment (A) and a hydrophobic segment (B). Such drugs as adriamycin or indomethacin may be physically encapsulated within the cores of the polymeric micelles formed from the block copolymer, so that the block copolymer micelles may be used as drug delivery systems. However, the polymeric micelles formed from the block copolymer cause many problems in the case of *in vivo* applications, since they cannot be hydrolyzed *in vivo* but are degraded only by enzymes, and they have poor biocompatibility because they cause immune responses, or the like.

Therefore, many attempts have been made to develop core-shell type drug delivery systems having improved biodegradability and biocompatibility.

For example, diblock or multiblock copolymers including polyalkylene glycol as a hydrophilic polymer and polylactic acid as a hydrophobic polymer are known to those skilled in the art. More particularly, acrylic acid derivatives are bonded to the end groups of such diblock or multiblock copolymers to form copolymers. The resultant copolymers are subjected to crosslinking to stabilize the polymeric micelles.

However, methods for preparing such diblock or multiblock copolymers have difficulties in introducing crosslinkers to the hydrophobic segments of A-B or A-B-A type diblock or triblock copolymers so that the polymers may form stable structures via crosslinking. Additionally, the crosslinkers used in the above methods cannot ensure safety in the human body because the crosslinkers have not been applied in the human body as yet. Furthermore, the crosslinked polymers cannot be degraded *in vivo*, and thus cannot be applied for *in vivo* use.

In addition to the above, known methods for preparing a polymeric micelle composition include an emulsification process, a dialysis process and a solvent evaporation process. The emulsification process includes dissolving polylatic acid into a water immiscible solvent, adding a drug to the polymer solution so that the drug is completely dissolved therein, further adding a surfactant thereto to form an oil-in-water emulsion, and evaporating the emulsion gradually under vacuum. Since the emulsification process requires a system for forming the emulsion, it is difficult and sophisticated to set the processing conditions. Additionally, since the emulsification process includes evaporation of an organic solvent, it requires a long period of processing time. Meanwhile, the dialysis process requires consumption of a large amount of water and needs a long period of processing time. Further, the solvent evaporation process requires a system such as a rotary vacuum distillator, for removal of solvent, and it takes a long period of time to remove the solvent completely. Moreover, the solvent evaporation process essentially includes a step of exposing pharmaceutically active ingredients to a high temperature for a long period of time, and thus it may cause such problems as degradation of pharmaceutically active ingredients or decreased pharmacological effects. Document US 2007/0003625 A1 relates to a branched polylactic acid derivative capable of forming polymeric micells in aqueous solution of pH 4 or more, and a preparation method and use thereof, wherein the branched polylactic acid derivative contains carboxy group or carboxy alkali metal salt group in the terminal of polymer chain.

Document WO 01/87345 A1 relates to a biocompatible stable composition containing a hydrophobic drug, such as paclitaxel. The composition, which forms a syringeable polymeric micellar solution in aqueous or body fluids, is a freeze-dried product comprising a hydrophobic drug, i.e. paclitaxel, and an amphiphilic block copolymer wherein a hydrophobic group having affinity or attraction with the hydrophobic drug, such as paclitaxel, is incorporated on its end.

Document WO 02/45689 A1 relates to a composition that comprises amphiphilic copolymers, poorly soluble drugs, biodegradable polymers (additional) and liquid PEG Micelles are formed by block copolymers in liquid PEG, and the drug is placed inside the micelles. The biodegradable polymer forms a matrix within the liquid PEG and forms an implant (trapezoid) when it is injected into human body. When the composition is injected into a human body, the liquid PEG is diluted by the body fluid, and the micelles within the matrix of biodegradable polymers are slowly released inside the human body.

Document WO 01/85216 A1 relates to a preparation method for and the pharmaceutical application of polymeric micelles. The micelle, which is used as a carrier for hydrophobic drugs, is prepared via phase separation of a biodegradable polymeric composition containing a block copolymer having a hydrophilic poly(alkylene glycol) component and a hydrophobic biodegradable polymer component suspended in a poly(ethylene glycol) medium.

Document US 2004/005351 A1 relates to cosolvent evaporation methods and compositions for improving the solubility of hydrophobic compounds, including therapeutic agents such as anticancer drugs, polyene antibiotics, antilipidemic agents, and hydrophobic compounds used in various industries, and/or for reducing the toxicity of certain hydrophobic therapeutic agents, especially polyene antibiotics, in particular, Amphotericin B (AmB), and therapeutics such as paclitaxel, tamoxifen, an acylated prodrug or an acylated cis-platin, by incorporating these agents within micelles comprising an amphiphilic block-forming copolymer.

## SUMMARY

Disclosed herein is a drug-containing polymeric micelle composition.

Disclosed herein too is a process for preparing the polymeric micelle composition.

## BRIEF DESCRIPTION OF THE DRAWINGS

The above and other aspects, features and advantages of the disclosed exemplary embodiments will be more apparent from the following detailed description taken in conjunction with the accompanying drawings in which:

FIG. 1 is the $^1$H NMR spectrum of the diblock copolymer [mPEG-PLA] obtained from Preparation Example 1; and
FIG. 2 is the $^1$H NMR spectrum of the diblock copolymer [mPEG-PLGA] obtained from Preparation Example 2.

## DETAILED DESCRIPTION

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein. Rather, these exemplary embodiments are provided so that this disclosure will be thorough and complete, and will fully convey the scope of this disclosure to those skilled in the art. In the description, details of well-known features and techniques may be omitted to avoid unnecessarily obscuring the presented embodiments.

The terminology used herein is for the purpose of describing particular embodiments only and is not intended to be limiting of this disclosure. As used herein, the singular forms "a", "an" and "the" are intended to include the plural forms as well, unless the context clearly indicates otherwise. Furthermore, the use of the terms a, an, etc. does not denote a limitation of quantity, but rather denotes the presence of at least one of the referenced item. It will be further understood that the terms "comprises" and/or "comprising", or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

In one aspect of the present invention, there is provided a process for preparing a polymeric micelle composition having poorly soluble drugs, which includes: dissolving a poorly soluble drug and an amphiphilic block copolymer into an organic solvent to obtain a solution; and adding an aqueous solution to the resultant solution to form polymeric micelles, wherein the process requires no separate operation to remove the organic solvent prior to the addition of aqueous solution, wherein the organic solvent is used in an amount of 0.5-15 wt%, based on the total weight of the composition, and wherein the aqueous solution comprises an osmolality adjusting agent which is at least one selected from a group consisting of sodium chloride, calcium chloride, sodium sulfate and magnesium chloride.

In another aspect of the present invention, there is provided a polymeric micelle composition in a form of lyophilized composition containing a poorly soluble drug, wherein said composition consists of a taxane, an amphiphilic block copolymer having a hydrophilic block and a hydrophobic block, an osmolality adjusting agent, and a lyophilization aid, wherein the osmolality adjusting agent is at least one selected from the group consisting of sodium chloride, calcium chloride, sodium sulfate and magnesium chloride.

The claimed process provided herein is simple, reduces the processing time, and is amenable to large-scale production. Additionally, the drug-containing polymeric micelle composition may be obtained at low temperature or room temperature, thereby improving the drug stability.

As mentioned above, the process for preparing a drug-containing polymeric micelle composition includes: dissolving a poorly soluble drug and an amphiphilic block copolymer into an organic solvent to obtain a solution; and adding an aqueous solution to the resultant solution to form polymeric micelles, wherein the process requires no separate operation to remove the organic solvent prior to the addition of aqueous solution, wherein the organic solvent is used in an amount of 0.5-15 wt%, based on the total weight of the composition, and wherein the aqueous solution comprises an osmolality adjusting agent which is at least one selected from a group consisting of sodium chloride, calcium chloride, sodium sulfate and magnesium chloride.

More particularly, to prepare the micelle composition, a poorly soluble drug and an amphiphilic block copolymer are dissolved in an organic solvent, particularly a water miscible organic solvent, used in an amount of 0.5-15wt% based on the total weight of the composition, and then an aqueous solution comprising an osmolality adjusting agent which is at least one selected from a group consisting of sodium chloride, calcium chloride, sodium sulfate and magnesium chloride is added thereto to form polymeric micelles in the organic solvent-aqueous solution mixure. Therefore, the polymeric micelle composition obtained from the claimed process provided herein includes a drug, an amphiphilic block copolymer, an osmolality adjusting agent as described above. In addition, the process requires no special operation to remove the organic solvent used for preparing polymer micelles prior to the formation of micelles.

The presence of an organic solvent in a micelle solution during the formation of micelles facilitates dissociation of micelles due to a high affinity of the hydrophobic portion of amphiphilic polymer micelles to the organic solvent, thereby accelerating precipitation of hydrophobic drug molecules. For this reason, processes for preparing polymer micelles known to date include dissolving a drug and an amphiphilic polymer into an organic solvent, removing the organic solvent, and adding an aqueous solution to form micelles. However, such processes need a long period of processing time to remove the organic solvent, and require an additional system, such as a reduced pressure distillator. In addition, the organic solvent may still remain partially in the reaction system even after removing it. Further, the drug may be degraded as it is exposed to high temperature for a long time during the removal of the organic solvent.

In one embodiment of the present invention, the process provided herein includes forming micelles at low temperature instead of removing the organic solvent at high temperature during the formation of micelles. In general, when polymeric micelles are heated, associated amphiphilic polymers become susceptible to dissociation as the unimers of amphiphilic polymer get an increased kinetic energy. As a result, hydrophobic drug molecules present in the hydrophobic core of micelles are easily in contact with the aqueous phase, thereby causing formation and precipitation of drug crystals. On the contrary, the process disclosed herein requires no separate solvent evaporation at high temperature, thereby reducing the processing step and preventing degradation of a drug. Further, the process disclosed herein is carried out at low temperature so that the resultant polymeric micelles maintain their stability.

In one embodiment of the claimed process, the polymeric micelles are formed by adding the aqueous solution as described above to the poorly soluble drug/amphiphilic polymer mixture in the organic solvent as described above at a temperature of 0-60°C, particularly 10-50°C, more particularly 10-40°C.

Although the organic solvent is not removed and exists at the certain concentration described above in the reaction mixture, the process disclosed herein does not allow precipitation of the drug because of the micelle formation at low temperature. More particularly, this is because the amphiphilic polymer and organic solvent molecules have a decreased kinetic energy under such a low temperature, and thus the drug present in the hydrophobic portions of amphiphilic polymer micelles may not be easily exposed to the aqueous phase.

In another embodiment of the claimed process, although there is no particular limitation in the type of poorly soluble drug encapsulated within the micelle structures of the amphiphilic block copolymer, the drug may be a poorly soluble drug having a solubility of 100 mg/mL or less to water. The process disclosed herein aims at a composition for administering a poorly soluble drug to the human body by encapsulating the drug within micelle structures.

In still another embodiment of the claimed process, the poorly soluble drug may be selected from anticancer agents. Particularly, the poorly soluble drug may be selected from taxane anticancer agents. Particular examples of taxane anticancer agents include paclitaxel, docetaxel, 7-epipaclitaxel, t-acetyl paclitaxel, 10-desacetylpaclitaxel, 10-desacetyl-7-epipaclitaxel, 7-xylosylpaclitaxel, 10-desacetyl-7-glutarylpaclitaxel, 7-N,N-dimethylglycylpaclitaxel, 7-L-alanylpaclitaxel or a mixture thereof. More particularly, the taxane anticancer agent may be paclitaxel or docetaxel.

In one embodiment of the claimed process, the amphiphilic block copolymer includes a diblock copolymer having a hydrophilic block (A) and a hydrophobic block (B) linked with each other in the form of A-B structure, and is non-ionic. Additionally, the amphiphilic block copolymer forms core-shell type polymeric micelles in its aqueous solution state, wherein the hydrophobic block (B) forms the core and the hydrophilic block (A) forms the shell.

In another embodiment of the claimed process, the hydrophilic block (A) of the amphiphilic block copolymer is a water soluble polymer, and includes at least one polymer selected from the group consisting of polyalkylene glycol, polyvinyl alcohol, polyvinylpyrrolidone, polyacrylamide and derivatives thereof. More particularly, the hydrophilic block (A) may be at least one polymer selected from the group consisting of monomethoxypolyalkylene glycol, monoacetoxypolyethylene glycol, polyethylene glycol, polyethylene-co-polypropylene glycol and polyvinylpyrrolidone. The hydrophilic block (A) may have a number average molecular weight of 500-50,000 daltons, more particularly 1,000-20,000 daltons.

The hydrophobic block (B) of the amphiphilic block copolymer is not dissolved in water and may be a biodegradable polymer with high biocompatibility. For example, the hydrophobic block (B) may be at least one polymer selected from the group consisting of polyester, polyanhydride, polyamino acid, polyorthoester, polyphosphazine and derivatives thereof. Particularly, the hydrophobic block (B) may be at least one polymer selected from the group consisting of polylactide, polyglycolide, polycaprolactone, polydioxan-2-one, polylactic-co-glycolide, polylactic-co-dioxan-2-one, polylactic-co-caprolactone and polyglycolic-co-caprolactone. More particularly, the hydrophobic block (B) may be polylactic acid or copolymer of polylactic acid and glycolic acid.

In addition, the above polymers listed as a hydrophobic block (B) may be provided as derivatives thereof substituted with fatty acid groups at the carboxy end groups. The fatty acid group may be at least one selected from the group consisting of butyrate, propionate, acetate, stearate and palmitate groups. The hydrophobic block (B) may have a number average molecular weight of 500-50,000 daltons, and more particularly 1,000-20,000 daltons.

In still another embodiment of the claimed process, to form stable polymeric micelles in an aqueous solution, the amphiphilic block copolymer includes the hydrophilic block (A) and the hydrophobic block (B) in a weight ratio of 3:7 to 8:2 (hydrophilic block (A) : hydrophobic block (B)), particularly of 4:6 to 7:3. When the proportion of the hydrophilic block (A) is lower than the above range, the polymer may not form polymeric micelles in an aqueous solution. On the other hand, when the proportion of the hydrophilic block (A) is higher than the above range, the polymer may be too hydrophilic to maintain its stability.

In still another embodiment, the organic solvent used in the claimed process is a water miscible organic solvent, and may be at least one solvent selected from the group consisting of alcohols, acetone, tetrahydrofuran, acetic acid, acetonitrile and dioxane. More particularly, the alcohol may be at least one alcohol selected from the group consisting of methanol, ethanol, propanol and butanol.

In still another embodiment, although the organic solvent is required to dissolve the polymer and the drug, the organic solvent is used in the claimed process in a small amount because the presence of the organic solvent decreases the micelle stability and accelerates drug precipitation. Thus the organic solvent is used in an amount of 0.5-15 wt%, based on the total weight of the composition from which the organic solvent is not removed. Indeed when the organic solvent is used in an amount less than 0.5 wt%, it is difficult to dissolve the drug in the organic solvent. On the other hand, when the organic solvent is used in an amount greater than 30 wt%, drug precipitation may occur during micellization or reconstitution.

The poorly soluble drug may be dissolved into the organic solvent sequentially or simultaneously with the polymer.

In the claimed process provided herein, the drug and the polymer may be simultaneously added to and dissolved in the organic solvent, or the polymer may be dissolved first into the organic solvent, followed by the drug, and vice versa. The drug and the polymer may be dissolved into the organic solvent at any temperature where the drug degradation may be prevented. The temperature may be 0-60°C, particularly 10-50°C, and more particularly 10-40°C.

A particular embodiment of the process for preparing a drug-containing polymeric micelle composition is claimed in claim 1.

The aqueous solution used in the claimed process may include water, distilled water, distilled water for injection, saline, 5% glucose, buffer, etc.

The polymeric micelle formation may be carried out by adding the aqueous solution at a temperature of 0-60°C, particularly 10-50°C, and more particularly 10-40°C.

In still another embodiment of the present invention, a lyophilization aid may be added to the micelle composition to perform lyophilization, after forming the polymeric micelles. The lyophilization aid may be added in order to allow a lyophilized composition to maintain its cake-like shape. In one embodiment of the lyophilized composition disclosed herein, the lyophilization aid may be at least one selected from the group consisting of sugar and sugar alcohols. The sugar may be at least one selected from lactose, maltose, sucrose, trehalose and a combination thereof. The sugar alcohol may be at least one selected from the mannitol, sorbitol, maltitol, xylitol, lactitol and a combination thereof.

In addition, the lyophilization aid serves to help the polymeric micelle composition to be dissolved homogeneously in a short time during the reconstitution of the lyophilized composition. In this context, the lyophilization aid may be used in an amount of 1-99.8 wt%, and more particularly 10-60 wt%, based on the total weight of the lyophilized composition.

In one embodiment, the claimed polymeric micelle composition may include 0.1-30 wt% of a poorly water soluble drug and 70-99.9 wt% of an amphiphilic block copolymer having a hydrophilic block and a hydrophobic block, based on the total dry weight of the composition.

The claimed process provided herein is simple, reduces the processing time, and is amenable to large-scale production, because it avoids a need for special operation to remove the organic solvent. Additionally, the drug-containing polymeric micelle composition may be obtained at low temperature or room temperature, thereby improving the drug stability.

According to the present invention, the aqueous solution further includes an osmolality adjusting agent. For example, the aqueous solution may include the osmolality adjusting agent at an osmolality of 30-15,000 mOsm/kg, particularly 100-5,000 mOsm/kg, and more particularly 200-2,500 mOsm/kg. When the osmolality is less than 30 mOsm/kg, it may not be desirable to prevent precipitation of the drug sufficiently during the preparation of the composition. On the other hand, when the osmolality is higher than 15,000 mOSm/kg, interlayer separation may occur in the polymer.

The osmolality adjusting agent functions to improve the stability of the poorly water soluble drug-containing polymeric micelle composition. Particularly, the osmolality adjusting agent significantly improves the stability of the composition in its aqueous solution state. A mechanism for the function of the osmolality adjusting agent is as follows: the degree of encapsulation of a drug within a polymeric micelle structure is in proportion to the fraction of cores formed from the hydrophobic block of the polymer in an aqueous solution. Additionally, the stability of the polymeric micelles depends on the dynamic equilibrium state formed by the polymeric micelles in an aqueous solution, i.e., on the equilibrium constant between the polymeric micelles and the individual polymer unimers dissolved in water.

Although a large amount of poorly soluble drug may be encapsulated within a polymeric micelle structure according to the claimed process, the hydrophilic blocks of the polymeric micelles may be surrounded with a great amount of water molecules upon the encapsulation of the drug, and thus the interaction between the water molecules and the hydrophilic blocks may weaken the hydrophobic interaction between hydrophobic blocks of the micelles, thereby destabilizing the micelles. Addition of the osmolality adjusting agent causes an electrostatic attraction force between the osmolality adjusting agent and water, resulting in dissociation of water molecules from the hydrophilic blocks of the polymeric micelles. As a result, the hydrophobic interaction between the hydrophobic blocks, which otherwise would participate in loose interaction, increases relatively, so that stable micelle structures may be formed. Therefore, addition of the osmolality adjusting agent may improve the stability of micelles. In other words, the stability of micelles increases during the process of preparing the micelle composition such as between the steps of adding the aqueous solution and the lyophilization,. In addition, the osmolality adjusting agent is not removed during the preparation of the composition disclosed herein but remains in the finished composition. Through the stabilization effect realized by the osmolality adjusting agent, the poorly water soluble drug-containing amphiphilic block copolymer micelle composition may have excellent stability. In one embodiment, by minimizing the use of organic solvent and comprising the osmolality adjusting agent, the composition wherein precipitation of a drug is prevented for 12 hours after reconstitution may be prepared.

The osmolality adjusting agent is pharmaceutically acceptable one and may be selected from any osmolality adjusting agents as long as it does not cause hemolysis upon the contact with blood. According to the present invention, the osmolality adjusting agent is an electrolyte such as an inorganic salt selected from the group consisting of sodium chloride, calcium chloride, sodium sulfate and magnesium chloride. Particularly, the osmolality adjusting agent may be sodium chloride or calcium chloride, and more particularly sodium chloride.

In still another aspect of the present invention, there is provided a polymeric micelle composition in a form of lyophilized composition containing a poorly soluble drug, wherein said composition consists of a taxane, an amphiphilic block copolymer having a hydrophilic block and a hydrophobic block, an osmolality adjusting agent, and a lyophilization aid, wherein the osmolality adjusting agent is at least one selected from the group consisting of sodium chloride, calcium chloride, sodium sulfate and magnesium chloride. In one embodiment, the composition may include 0.1-30 wt% of taxane, 20-99 wt%, particularly 60-99 wt% of an amphiphilic block copolymer having a hydrophilic block and a hydrophobic block, and 0.1-50 wt% of the osmolality adjusting agent, based on the total dry weight of the composition. In one embodiment, the composition may include 0.1-50 wt%, particularly 0.5-20 wt%, and more particularly 1-10 wt% of the osmolality adjusting agent, based on the total dry weight of the composition. Detailed description about the types and amounts of the poorly soluble drug, amphiphilic block copolymer and osmolality adjusting agent is the same as described above.

The composition disclosed herein may further include pharmaceutical adjuvants and other therapeutically useful substances, such as a preservative, stabilizer, hydrating agent, emulsifier, salt for adjusting osmotic pressure and/or buffer. The composition may be formulated into various types of oral or parenteral formulations according to a manner generally known to those skilled in the art.

Typical examples of the parenteral formulations include injection formulations in the form of suspension. Formulations for parenteral administration may be administered via a rectal, local, transdermal, intravenous, intramuscular, intraperitoneal or subcutaneous route. In example embodiment, the composition may be provided in a lyophilized form, which is to be reconstituted with distilled water for injection, 5% glucose, 5% saline, etc., so that it may be administered via intravascular injection. In one embodiment, the composition, which is reconstituted with aqueous solution, may include 0.1-15 wt% of the poorly soluble drug. In addition, the composition includes 10-150 mg/ml of an amphiphilic block copolymer, 5-30 mg/ml, particularly 10-20 mg/ml of an osmolality adjusting agent, and 1-100 mg/ml of a lyophilization aid. In another embodiment, the lyophilized composition may regulate a particle size of micelles in the range of 1-400 nm, particularly 5-200 nm, according to molecular weights of the copolymer in aqueous solution.

Formulations for oral administration include tablets, pills, hard and soft capsules, liquid, suspension, emulsion, syrup, granules, etc. Such formulations may include a diluent (e.g. lactose, dextrose, sucrose, mannitol, sorbitol, cellulose and glycine), a lubricant (e.g. silica, talc, stearic acid and magnesium or calcium salts thereof, as well as polyethylene glycol), etc. in addition to active ingredients. Tablets may include binders, such as magnesium aluminum silicate, starch paste, gelatin, tragacanth, methylcellulose, sodium carboxymethylcellulose and polyvinylpyrrolidine. Optionally, tablets may include pharmaceutically acceptable additives including disintegrating agents, absorbing agents, coloring agents, flavoring agents and sweetening agents, such as starch, agar, alginic acid or sodium salt thereof. Tablets may be obtained by a conventional mixing, granulating or coating process.

## EXAMPLES

The examples and experiments will now be described. The following examples and experiments are for illustrative purposes only and not intended to limit the scope of this disclosure.

The amphiphilic block copolymer used in the composition and process disclosed herein is obtained according to a method as described in International Patent Publication No. WO 03/33592 that relates to a polymeric micelle composition, and more specifically, to a polymeric micelle composition comprising an amphiphilic block copolymer composed of a hydrophilic block and a hydrophobic block, and a polylactic acid derivative having at least one terminal carboxyl group.

Preparation Example 1: Synthesis of Monomethoxypolyethylene Glycol-Polylactide (mPEG-PLA) Block Copolymer (A-B Type)

First, 5.0 g of monomethoxypolyethylene glycol (number average molecular weight: 2,000 daltons) was introduced into a 100 mL two-neck round-bottom flask, and was heated to 130°C under reduced pressure (1 mmHg) for 3-4 hours to remove water therefrom. Next, the flask was purged with nitrogen gas, and stannous octoate $(Sn(Oct)_2)$ was added thereto as a reaction catalyst using a syringe in an amount of 0.1 wt% (10.13 mg, 25 mmol) based on the weight of D, L-lactides. After the reaction mixture was agitated for 30 minutes, it was subjected to depressurization (1 mmHg) at 130°C for 1 hour to remove the solvent (toluene) in which the catalyst is dissolved. Then, 10.13 g of purified lactide was added thereto, and the resultant mixture was heated at 130°C for 18 hours. After heating, the resultant polymer was dissolved into methylene chloride, and was added to diethyl ether to cause precipitation of the polymer. The resultant polymer was dried in a vacuum oven for 48 hours.

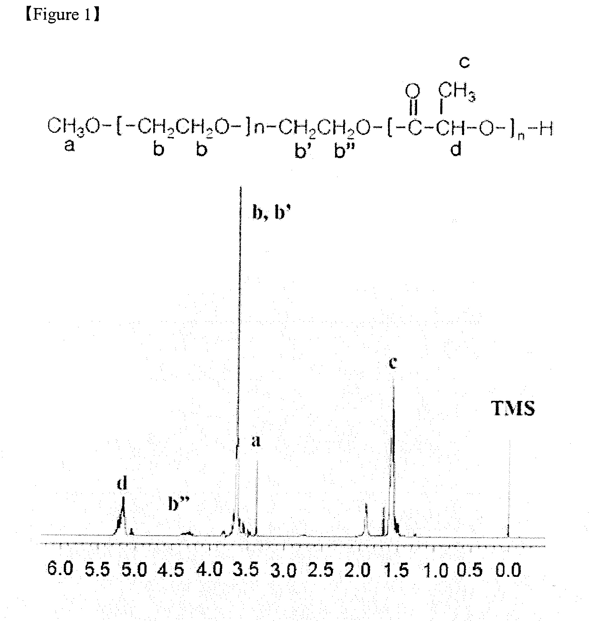

The copolymer, monomethoxylpolyethylene glycol-polylactide (mPEG-PLA) has a number average molecular weight of 2,000-1,765 daltons. After analyzing the copolymer by $^1$H-NMR, it was shown that the copolymer is an A-B type diblock copolymer (see Fig. 1).

Preparation Example 2: Synthesis of Monomethoxypolyethylene Glycol-Poly(lactic-co-glycolide) (mPEG-PLGA) Block Copolymer (A-B Type)

A block copolymer was obtained by reacting monomethoxypolyethylene glycol (number average molecular weight: 5,000 daltons), lactide and glycolide in the presence of stannous octoate as a catalyst at 120°C for 12 hours in the same manner as described in Preparation Example 1.

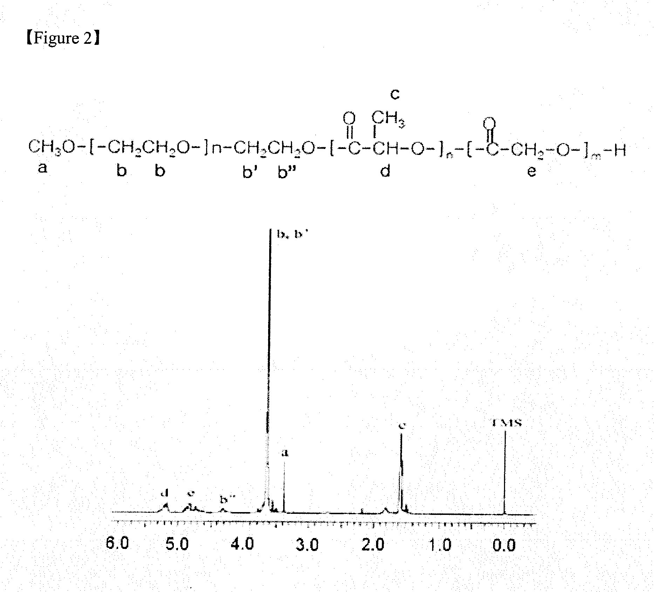

The copolymer, monomethoxypolyethylene glycol-poly(lactic-co-glycolide) (mPEG-PLGA) has a number average molecular weight of 5,000-4,000 daltons and is an A-B type copolymer. After analyzing the copolymer by $^1$H-NMR, it was shown that the copolymer is an A-B type diblock copolymer (see Fig. 2).

Examples 1-4 Preparation of Polymeric Micelle Compositions Containing Docetaxel are not part of the claimed invention

The amphiphilic block copolymer, monomethoxypolyethylene glycol-polylactide (number average molecular weight: 2,000-1,765 daltons), obtained from Preparation Example 1 was used. The amphiphilic block copolymer was completely dissolved at 60°C in the amount as described in the following Table 1, and 0.08 mL of ethanol was added thereto, followed by mixing. Next, the resultant mixture was cooled to 30°C, docetaxel was added thereto, and the mixture was agitated until a clear solution containing docetaxel completely dissolved therein was obtained. Then, the solution was cooled to 25°C, and 4.0 mL of purified water was added thereto at room temperature, and the reaction mixture was allowed to react until a bluish clear solution was formed, thereby forming polymeric micelles. Then, 100 mg of D-mannitol was completely dissolved into the solution as a lyophilizing agent, and the resultant solution was filtered through a filter with a pore size of 200 nm, followed by lyophilization, to obtain a powdery docetaxel-containing polymeric micelle composition.

[Table 1]

| | | Amount (mg) | |
|---|---|---|---|
| | | Docetaxel | Amphiphilic Block Copolymer |
| Example 1 | | 20.0 | 380.0 |
| Example 2 | | 20.0 | 265.0 |
| Example 3 | | 20.0 | 180.0 |
| Example 4 | | 20.0 | 760.0 |

Examples 5-7: Preparation of Polymeric Micelle Compositions Containing Paclitaxel are not part of the claimed invention

The amphiphilic block copolymer, monomethoxypolyethylene glycol-polylactide (number average molecular weight: 2,000-1,765 daltons), obtained from Preparation Example 1 was used. The amphiphilic block copolymer was completely dissolved at 80°C in the amount as described in the following Table 2, and ethanol was added thereto, followed by mixing. Next, paclitaxel was added to the mixture, and the resultant mixture was agitated until a clear solution containing paclitaxel completely dissolved therein was obtained. Then, the solution was cooled to 50°C, 5.0 mL of purified water was added thereto at room temperature, and the reaction mixture was allowed to react until a bluish clear solution was formed, thereby forming polymeric micelles. Then, 100 mg of anhydrous lactose was completely dissolved into the solution as a lyophilizing agent, and the resultant solution was filtered through a filter with a pore size of 200 nm, followed by lyophilization, to obtain a powdery paclitaxel-containing polymeric micelle composition.

[Table 2]

| | | Amount (mg) | | |
|---|---|---|---|---|
| | | Paclitaxel | Amphiphilic Block Copolymer | Ethanol |
| Example 5 | | 30.0 | 570.0 | 95.5 (0.121 mL) |
| Example 6 | | 30.0 | 270.0 | 45.0 (0.057 mL) |
| Example 7 | | 30.0 | 170.0 | 28.4 (0.036 mL) |

Example 8-10: Preparation of mPEG-PLA Block Copolymer Micelle Composition Containing Sodium Chloride and Docetaxel

First, 360 mg or 760 mg of the amphiphilic block copolymer, mPEG-PLA (number average molecular weight: 2,000-1,765 daltons), obtained from Preparation Example 1 was completely dissolved into 0.2 mL of ethanol at 60°C to provide a clear ethanol solution including the copolymer. The ethanol solution was cooled to 25°C, and 20 mg of docetaxel was added thereto and the resultant solution was agitated until docetaxel is completely dissolved.
Next, aqueous solutions each containing 0.9 wt% and 1.8 wt% of sodium chloride and having an osmolality of 300 mOsm/kg and 600 mOsm/kg respectively were prepared in separate containers. The osmolality was measured by using a commercially available osmometer (Gonotech GmbH, OSMOMAT030). Each aqueous solution was added to the ethanol solution including the copolymer in an amount of 4 mL, and the resultant mixture was agitated at 40°C for 10 minutes to form an aqueous polymeric micelle solution.
Then, 100 mg of D-mannitol was dissolved into each solution, and the resultant solution was filtered through a filter with a pore size of 200 nm to remove undissolved docetaxel, followed by lyophilization.
The lyophilized composition was subjected to liquid chromatography as follows to determine the content of docetaxel. Additionally, particle size was measured by a dynamic light scattering (DLS) method. The results are shown in the following Table 3.

[Table 3]

| | mPEG-PLA (mg) | Docetaxel (mg) | NaCl (mg) (Osmolality (mOsm/Kg)) | Docetaxel Content (wt%) | Particle Size (nm) |
|---|---|---|---|---|---|
| Example 8 | 380 | 20 | 36(300) | 99.8 | 20 |

(continued)

|  | mPEG-PLA (mg) | Docetaxel (mg) | NaCl (mg) (Osmolality (mOsm/Kg)) | Docetaxel Content (wt%) | Particle Size (nm) |
|---|---|---|---|---|---|
| Example 9 | 760 | 20 | 36 (300) | 99.9 | 18 |
| Example 10 | 760 | 20 | 72 (600) | 99.8 | 19 |

Liquid Chromatography

1) Column: a stainless steel column having a length of 250 mm and an inner diameter of 4.6 mm and packed with pentafluorophenyl-coated particles having a particle diameter of 5 $\mu$m and a pore diameter of 300Å.
2) Mobile Phase: acetonitrile: methanol: water = 26:32:42
3) Flow Rate: 1.5 mL/min.
4) Loading Amount: 20 $\mu$L
5) Detector: UV absorption spectrometer (wavelength measurement: 232 nm)

Example 11: Preparation of mPEG-PLA Block Copolymer Micelle Composition Containing Calcium Chloride and Paclitaxel

First, 100 mg of the amphiphilic block copolymer, mPEG-PLA (number average molecular weight: 2,000-1,765 daltons), obtained from Preparation Example 1 was completely dissolved into 0.1 mL of ethanol at 60°C to provide a clear ethanol solution including the copolymer. The ethanol solution was cooled to 25°C, and 20 mg of paclitaxel was added thereto and the resultant solution was agitated until paclitaxel was completely dissolved.
Next, aqueous solutions each containing 0.9 wt% and 1.8 wt% of calcium chloride and having an osmolality of 230 mOsm/kg and 460 mOsm/kg respectively were prepared in separate containers. Each aqueous solution was added to the ethanol solution including the copolymer in an amount of 4 mL, and the resultant mixture was agitated at 40°C for 10 minutes to form an aqueous polymeric micelle solution.
Then, 39 mg of D-mannitol was dissolved into each solution, and the resultant solution was filtered through a filter with a pore size of 200 nm to remove undissolved paclitaxel, followed by lyophilization.
The lyophilized composition was subjected to the liquid chromatography as described in Example 1 to determine the content of paclitaxel. Additionally, particle size was measured by a DLS method. The results are shown in the following Table 4.

[Table 4]

| mPEG-PLA (mg) | Paclitaxel (mg) | CaCl$_2$ (mg) (Osmolality (mOsm/Kg)) | Paclitaxel Content (wt%) | Particle Size (nm) |
|---|---|---|---|---|
| 100 | 20 | 36 (230) | 99.5 | 24 |
| 100 | 20 | 72(460) | 99.8 | 24 |

Example 12: Preparation of mPEG-PLGA Block Copolymer Micelle Composition Containing Sodium Chloride and Docetaxel

First, 760 mg of the amphiphilic block copolymer, mPEG-PLGA (number average molecular weight: 5,000-4,000 daltons), obtained from Preparation Example 2 was completely dissolved into 0.2 mL of acetone at 50°C to provide a clear acetone solution including the copolymer. The acetone solution was cooled to 25°C, and 40 mg of docetaxel was added thereto and the resultant solution was agitated until docetaxel was completely dissolved.
Next, 8 mL of an aqueous solution containing 0.9 wt% of sodium chloride and having an osmolality of 300 mOsm/kg was added to the drug-containing acetone solution including the copolymer, and the resultant mixture was agitated at 25°C for 20 minutes to form a homogeneous solution. Once the homogeneous solution was formed, 200 mg of D-mannitol was dissolved into the solution to provide a clear aqueous polymeric micelle solution. Finally, the aqueous polymeric micelle solution was filtered through a filter with a pore size of 200 nm to remove undissolved docetaxel, followed by lyophilization.

The lyophilized composition was subjected to the liquid chromatography as described in Example 1 to determine the content of docetaxel. Additionally, particle size was measured by a DLS method.
Docetaxel Content: 101. 3 wt%
Particle Size: 35 nm

Example 13: Preparation of mPEG-PLGA Block Copolymer Micelle Composition Containing Calcium Chloride and Paclitaxel

First, 100 mg of the amphiphilic block copolymer, mPEG-PLGA (number average molecular weight: 5,000-4,000 daltons), obtained from Preparation Example 2 was completely dissolved into 0.2 mL of acetone at 50°C to provide a clear acetone solution including the copolymer. The acetone solution was cooled to 25°C, and 40 mg of paclitaxel was added thereto and the resultant solution was agitated until paclitaxel was completely dissolved.

Next, 8 mL of an aqueous solution containing 0.9 wt% of calcium chloride and having an osmolality of 230 mOsm/kg was added to the drug-containing acetone solution including the copolymer, and the resultant mixture was agitated at 25°C for 20 minutes to form a homogeneous solution. Once the homogeneous solution was formed, 53 mg of D-mannitol was dissolved into the solution to provide a clear aqueous polymeric micelle solution. Finally, the aqueous polymeric micelle solution was filtered through a filter with a pore size of 200 nm to remove undissolved paclitaxel, followed by lyophilization.

The lyophilized composition was subjected to high-performance liquid chromatography (HPLC) to determine the content of paclitaxel. Additionally, particle size was measured by a DLS method.
Paclitaxel Content: 101. 1 wt%
Particle Size: 35 nm.

Comparative Example 1: Preparation of Docetaxel-Containing Polymeric Micelles Using Solvent Evaporation Process

First, docetaxel and the amphiphilic block copolymer were provided in the same amounts as described in Example 3. Next, 5 mL of ethanol was added to docetaxel and the amphiphilic block copolymer and the resultant mixture was agitated at 60°C until the materials were completely dissolved to obtain a clear solution. Then, ethanol was distilled off under reduced pressure at 60°C for 3 hours using a rotary vacuum distillator equipped with a round-bottom flask. The reaction mixture was cooled to 25°C, 4 mL of purified water at room temperature was added thereto and the reaction mixture was allowed to react until a bluish clear solution was obtained, thereby forming polymeric micelles. Then, 100 mg of D-mannitol as a lyophilizing agent was added to the polymeric micelles so that the micelles are completely dissolved, and the resultant mixture was filtered through a filter with a pore size of 200 nm, followed by lyophilization, thereby providing a powdery docetaxel-containing polymeric micelle composition.

Comparative Example 2: Preparation of Paclitaxel-Containing Polymeric Micelles Using Solvent Evaporation Process

First, paclitaxel and the amphiphilic block copolymer were provided in the same amounts as described in Example 7. Next, 5 mL of ethanol was added to paclitaxel and the amphiphilic block copolymer and the resultant mixture was agitated at 60°C until the materials were completely dissolved to obtain a clear solution. Then, ethanol was distilled off under reduced pressure at 60°C for 3 hours using a rotary vacuum distillator equipped with a round-bottom flask. The reaction mixture was cooled to 50°C, 5 mL of purified water at room temperature was added thereto and the reaction mixture was allowed to react until a bluish clear solution was obtained, thereby forming polymeric micelles. Then, 100 mg of anhydrous lactose as a lyophilizing agent was added to the polymeric micelles so that the micelles are completely dissolved, and the resultant mixture was filtered through a filter with a pore size of 200 nm, followed by lyophilization, thereby providing a powdery paclitaxel-containing polymeric micelle composition.

Comparative Example 3: Preparation of Micelles at High Temperature

A docetaxel-containing polymeric micelle composition was prepared by using the same amount of ingredients as described in Example 1, except that the polymeric micelles were formed while maintaining the temperature at 70°C after adding the ethanol solution. After that, the micelles were lyophilized in the same manner as described in Example 1 to obtain a lyophilized micelle composition.

Comparative Example 4: Preparation of Micelles at High Temperature

A paclitaxel-containing polymeric micelle composition was prepared by using the same amount of ingredients as described in Example 7, except that the polymeric micelles were formed while maintaining the temperature at 70°C after adding the ethanol solution. After that, the micelles were lyophilized in the same manner as described in Example 7 to obtain a lyophilized micelle composition.

**TEST EXAMPLES**

Test Example 1: Measurement of Amount of Drug Encapsulation

The docetaxel-containing polymeric micelle compositions according to Examples 1-4 and Comparative Example 1 were subjected to HPLC as specified in the following Table 5 to measure the concentration of docetaxel in each composition. Then, the drug content (encapsulation amount) was calculated according to the following Equation 1. The results are shown in the following Table 6.

[Equation 1]

$$\text{Encapsulation (\%)} = (\text{measured amount of docetaxel / amount of used docetaxel}) \times 100$$

[Table 5]

| | Condition |
|---|---|
| Mobile Phase | 45% Acetonitrile/55% Water |
| Column | C18, 300A Inner Diameter 4.6 mm, Length 25 cm (Phenomenex, USA) |
| Detection Wavelength | 227 nm |
| Flow Rate | 1.5 mL/min. |
| Temperature | Room Temperature |
| Injection Volume | 10 $\mu$L |

[Table 6]

| | Docetaxel Content (%) |
|---|---|
| Example 1 | 98.7 |
| Example 2 | 101.0 |
| Example 3 | 99.6 |
| Example 4 | 100.3 |
| Comparative Example 1 | 57.9 |

As can be seen from the above results, the compositions obtained after lyophilization without removing the organic solvent according to Examples 1-4 showed a docetaxel content of about 100%. On the other hand, the lyophilized composition obtained after removing the organic solvent according to Comparative Example 1 showed a docetaxel content of about 60%. This demonstrates that docetaxel was degraded during the evaporation of the organic solvent at high temperature in case the polymeric micelles was obtained via a solvent evaporation process according to Comparative Example 1.

Test Example 2: Measurement of Particle Size

The paclitaxel-containing polymeric micelle compositions according to Examples 5-7 and Comparative Example 2 were reconstituted with 5 mL of saline, and the particle size in each reconstituted composition was measured in aqueous solution using a particle size analyzer (DLS). The results are shown in the following Table 7.

[Table 7]

|  | Particle Size (nm) |
| --- | --- |
| Example 5 | 29.9 |
| Example 6 | 30.5 |
| Example 7 | 34.3 |
| Comparative Example 2 | 32.3 |

As can be seen from the above results, there is no significant difference in the particle size in aqueous solution among the lyophilized compositions obtained without removing the organic solvent according to Examples 5-7 and the lyophilized composition obtained after removing the organic solvent according to Comparative Example 2.

Test Example 3: Stability Test of Polymeric Micelle Aqueous Solution

For aqueous solutions of polymeric micelle compositions(docetaxel 5 mg/ml) of Example 8 and Example 1 before adding the lyophilization aid, while the solutions were left at 15 °C, starting time for precipitation of drug was measured.

[Table 8]

|  | Starting time for precipitation of drug(hr) |
| --- | --- |
| Example 8 | 4.5 |
| Example 1 | 2.5 |

As can be seen from the above results, due to the delayed starting time of precipitation, a stability of the micelle composition increases in polymeric micelle aqueous solution according to Example 8 containing sodium chloride as osmolality adjusting agent relative to aqueous solution according to Example 1.

Test Example 4: Stability Test

The paclitaxel-containing polymeric micelle composition according to Example 7 was compared with the paclitaxel-containing polymeric micelle composition according to Comparative Example 2 in terms of the stability in the aqueous solution at 37°C.

Each of the compositions according to Example 7 and Comparative Example 2 was diluted with distilled water for injection to a paclitaxel concentration of 1 mg/mL. While each diluted solution was left at 37°C, concentration of paclitaxel contained in each micelle structure was measured over time by way of HPLC. HPLC was carried out under the same conditions as described in the above Table 5. The results are shown in the following Table 9.

[Table 9]

| Time (hr) | Paclitaxel Concentration (mg/mL) | |
| --- | --- | --- |
|  | Example 7 | Comparative Example 2 |
| 0 | 1.00 | 1.00 |
| 2 | 1.01 | 0.99 |
| 4 | 0.99 | 0.99 |
| 8 | 0.98 | 0.99 |
| 12 | 1.00 | 0.98 |
| 24 | 0.99 | 0.99 |

As can be seen from the above results, there is no significant difference in the stability in aqueous solution over 24 hours between the lyophilized composition obtained without removing the organic solvent according to Example 7 and the lyophilized composition obtained after removing the organic solvent according to Comparative Example 2.

In addition, the lyophilized composition containing docetaxel and sodium chloride according to Example 9 and 10 was evaluated in terms of the stability in the aqueous solution at 37°C.

The lyophilized composition was diluted with distilled water for injection to a docetaxel concentration of 1 mg/mL. While each diluted solution was left at 37°C, concentration of docetaxel contained in each micelle structure was measured over time. The results are shown in the following Table 10.

[Table 10]

|  | mPEG-PLA (mg) | Docetaxel (mg) | NaCl (mg) (Osmolality (mOsm/Kg)) | Initial Docetaxel Concentration (mg/mL) | Docetaxel Concentration After 12 Hours (mg/mL) |
|---|---|---|---|---|---|
| Example 9 | 760 | 20 | 36 (300) | 1.0 | 0.95 |
| Example 10 | 760 | 20 | 72 (600) | 1.0 | 0.99 |
| Example 4 | 760 | 20 | 0 | 1.0 | 0.55 |

As can be seen from Table 10, stability of the polymeric micelle composition increases as the amount of the inorganic salt relative to the amphiphilic block copolymer increases. The stability of the polymeric micelle composition containing osmolality adjusting agent was improved, compared to the polymeric micelle composition not containing it.

Test Example 5

The docetaxel-containing polymeric micelle compositions according to Example 1 and Comparative Example 3 were compared with each other in terms of the docetaxel content and related compound content. The docetaxel content and the related compound content were measured under the same HPLC conditions as described in the above Table 5 and the following Table 11, respectively. The results are shown in the following Table 12.

[Table 11]

|  | Condition | | |
|---|---|---|---|
| Mobile Phase | Time(min.) | Water: Acetonitrile | |
|  | 0-15 | 65:35→35:65 | |
|  | 15-25 | 35:65→25:75 | |
|  | 25-30 | 25:75→5:95 | |
|  | 30-35 | 5:95→0:100 | |
|  | 35-39 | 0:100 | |
|  | 39-40 | 0:100→65:35 | |
|  | 40-45 | 65:35 | |
| Column | C18, 300A Inner Diameter 4.6 mm, Length 25 cm (Phenomenex, USA) | | |
| Detection Wavelength | 230 nm | | |
| Flow Rate | 1.0 mL/min. | | |
| Temperature | Room Temperature | | |
| Injection Volume | 10 μL | | |

[Table 12]

| Time | Content (%) | |
|---|---|---|
| | Docetaxel | Total related compounds |
| Example 1 | 98.7 | 0.97 |
| Comparative Example 3 | 88.9 | 5.44 |

As can be seen from the above results, high-temperature preparation causes an increase in the amount of docetaxel-related compounds to five times of the amount of those compounds in the case of low-temperature preparation, resulting in a drop in the docetaxel content. This means that high-temperature processing conditions cause degradation of a drug.

Test Example 7

The paclitaxel-containing polymeric micelle composition according to Example 7 was compared with the paclitaxel-containing polymeric micelle composition according to Comparative Example 4 in terms of the paclitaxel content. The paclitaxel content was measured under the same HPLC conditions as described in the above Table 5. Then, the drug content (encapsulation amount) was calculated according to the following Equation 2. The results are shown in the following Table 13.

[Equation 2]

$$\text{Encapsulation (\%)} = [\text{measured amount of paclitaxel / amount of used paclitaxel}] \times 100$$

[Table 13]

| | Paclitaxel content (%) |
|---|---|
| Example 7 | 99.6 |
| Comparative Example 4 | 59.9 |

The polymeric micelle composition obtained by adding water to form micelles in the presence of the organic solvent while maintaining a high temperature of 70°C according to Comparative Example 4 caused precipitation of the drug, paclitaxel. Particularly, the paclitaxel content in Comparative Example 4 was decreased as compared to the paclitaxel content in Example 7 by 40% or more.

While the exemplary embodiments have been shown and described, it will be understood by those skilled in the art that various changes in form and details may be made thereto without departing from the spirit and scope of this disclosure as defined by the appended claims.

In addition, many modifications can be made to adapt a particular situation or material to the teachings of this disclosure without departing from the essential scope thereof. Therefore, it is intended that this disclosure not be limited to the particular exemplary embodiments disclosed as the best mode contemplated for carrying out this disclosure, but that this disclosure will include all embodiments falling within the scope of the appended claims.

**Claims**

1. A method for preparing a polymeric micelle composition having poorly soluble drugs, comprising:

dissolving a poorly soluble drug and an amphiphilic block copolymer into an organic solvent to obtain a solution; and
adding an aqueous solution to the resultant solution to form polymeric micelles,

wherein the method requires no separate operation to remove the organic solvent prior to the addition of aqueous solution,

wherein the organic solvent is used in an amount of 0.5-15 wt%, based on the total weight of the composition, and wherein the aqueous solution comprises an osmolality adjusting agent which is at least one selected from a group consisting of sodium chloride, calcium chloride, sodium sulfate and magnesium chloride.

2. The method of claim 1, wherein the polymeric micelles are formed by adding the aqueous solution to the resultant solution at 0-60°C.

3. The method of claims 1 or 2, wherein the drug has a solubility of 100 mg/ml or less in water.

4. The method of any one of claims 1 to 3, wherein the amphiphilic block copolymer is a diblock copolymer having a hydrophilic block (A) and a hydrophobic block (B), wherein

the hydrophilic block (A) is at least one polymer selected from the group consisting of polyalkylene glycol, polyvinyl alcohol, polyvinylpyrrolidone, polyacrylamide and derivatives thereof, and

the hydrophobic block (B) is at least one polymer selected from the group consisting of polyester, polyanhydride, polyamino acid, polyorthoester, polyphosphazine and derivatives thereof.

5. The method of claim 4, wherein the amphiphilic block copolymer includes the hydrophilic block (A) and the hydrophobic block (B) in a weight ratio of 3:7 to 8:2 (hydrophilic block (A) : hydrophobic block (B)).

6. The method of any one of claims 1 to 5, wherein the organic solvent is at least one solvent selected from the group consisting of alcohols, acetone, tetrahydrofuran, acetic acid, acetonitrile and dioxane.

7. The method of any one of claims 1 to 6, wherein the aqueous solution includes the osmolality adjusting agent at an osmolality of 30-15,000 mOsm/kg.

8. The method of any one of claims 1 to 7, wherein a lyophilization aid is added to the micelle composition to perform lyophilization, after forming the polymeric micelles.

9. The method of any one of claims 1 to 8, wherein the polymeric micelle composition having poorly soluble drugs includes 0.1-30 wt% of a poorly soluble drug and 70-99.9 wt% of an amphiphilic block copolymer having a hydrophilic block and a hydrophobic block, based on the total dry weight of the composition.

10. A polymeric micelle composition in a form of lyophilized composition containing a poorly soluble drug, wherein said composition consists of a taxane, an amphiphilic block copolymer having a hydrophilic block and a hydrophobic block, an osmolality adjusting agent, and a lyophilization aid, wherein the osmolality adjusting agent is at least one selected from the group consisting of sodium chloride, calcium chloride, sodium sulfate and magnesium chloride.

11. The composition of claim 10, wherein the composition includes 0.1-30 wt% of taxane, 20-99 wt% of an amphiphilic block copolymer having a hydrophilic block and a hydrophobic block, and 0.1-50 wt% of an osmolality adjusting agent, based on the total dry weight of the composition.

12. The composition of claims 10 or 11, wherein the taxane includes paclitaxel, docetaxel, 7-epipaclitaxel, t-acetyl paclitaxel, 10-desacetylpaclitaxel, 10-desacetyl-7-epipaclitaxel, 7-xylosylpaclitaxel, 10-desacetyl-7-glutarylpaclitaxel, 7-N,N-dimethylglycylpaclitaxel, 7-L-alanylpaclitaxel or a mixture thereof.

13. The composition of any one of claims 10 to 12, wherein the hydrophilic block (A) is polyethylene glycol or monomethoxypolyethylene glycol, and the hydrophobic block (B) is polylactic acid or copolymer of polylactic acid and glycolic acid.

**Patentansprüche**

1. Verfahren zum Herstellen einer polymeren Mizellenzusammensetzung mit schlecht löslichen Arzneimitteln, umfassend:

Lösen eines schlecht löslichen Arzneimittels und eines amphiphilen Blockcopolymers in einem organischen Lösungsmittel unter Erhalt einer Lösung; und

Zugeben einer wässrigen Lösung zu der erhaltenen Lösung unter Bildung polymerer Mizellen,

wobei das Verfahren vor der Zugabe von wässriger Lösung keinen separaten Vorgang erfordert, um das organische Lösungsmittel zu entfernen,

wobei das organische Lösungsmittel in einer Menge von 0,5-15 Gew.-%, bezogen auf das Gesamtgewicht der Zusammensetzung, verwendet wird und

wobei die wässrige Lösung ein Mittel zum Einstellen der Osmolalität umfasst, bei dem es sich um mindestens eines handelt, das ausgewählt ist aus einer Gruppe bestehend aus Natriumchlorid, Calciumchlorid, Natriumsulfat und Magnesiumchlorid.

2. Verfahren nach Anspruch 1, wobei die polymeren Mizellen durch Zugeben der wässrigen Lösung zu der erhaltenen Lösung bei 0-60 °C gebildet werden.

3. Verfahren nach Anspruch 1 oder 2, wobei das Arzneimittel in Wasser eine Löslichkeit von 100 mg/ml oder weniger aufweist.

4. Verfahren nach einem der Ansprüche 1 bis 3, wobei es sich bei dem amphiphilen Blockcopolymer um ein Diblockcopolymer mit einem hydrophilen Block (A) und einem hydrophoben Block (B) handelt, wobei
es sich bei dem hydrophilen Block (A) um mindestens ein Polymer handelt, das ausgewählt ist aus der Gruppe bestehend aus Polyalkylenglycol, Polyvinylalkohol, Polyvinylpyrrolidon, Polyacrylamid und Derivaten davon, und
es sich bei dem hydrophoben Block (B) um mindestens ein Polymer handelt, das ausgewählt ist aus der Gruppe bestehend aus Polyester, Polyanhydrid, Polyaminosäure, Polyorthoester, Polyphosphazen und Derivaten davon.

5. Verfahren nach Anspruch 4, wobei das amphiphile Blockcopolymer den hydrophilen Block (A) und den hydrophoben Block (B) in einem Gewichtsverhältnis von 3:7 bis 8:2 (hydrophiler Block (A): hydrophober Block (B)) beinhaltet.

6. Verfahren nach einem der Ansprüche 1 bis 5, wobei es sich bei dem organischen Lösungsmittel um mindestens ein Lösungsmittel handelt, das ausgewählt ist aus der Gruppe bestehend aus Alkoholen, Aceton, Tetrahydrofuran, Essigsäure, Acetonitril und Dioxan.

7. Verfahren nach einem der Ansprüche 1 bis 6, wobei die wässrige Lösung das Mittel zum Einstellen der Osmolalität mit einer Osmolalität von 30-15,000 mOsm/kg beinhaltet.

8. Verfahren nach einem der Ansprüche 1 bis 7, wobei nach dem Bilden der polymeren Mizellen eine Lyophilisierungshilfe zu der Mizellenzusammensetzung gegeben wird, um eine Lyophilisierung durchzuführen.

9. Verfahren nach einem der Ansprüche 1 bis 8, wobei die polymere Mizellenzusammensetzung mit schlecht löslichen Arzneimitteln zu 0,1-30 Gew.-% ein schlecht lösliches Arzneimittel und zu 70-99,9 Gew.-% ein amphiphiles Blockcopolymer mit einem hydrophilen Block und einem hydrophoben Block, bezogen auf das Gesamttrockengewicht der Zusammensetzung, beinhaltet.

10. Polymere Mizellenzusammensetzung in Form einer lyophilisierten Zusammensetzung, die ein schlecht lösliches Arzneimittel enthält, wobei die Zusammensetzung aus einem Taxan, einem amphiphilen Blockcopolymer mit einem hydrophilen Block und einem hydrophoben Block, einem Mittel zum Einstellen der Osmolalität und einer Lyophilisierungshilfe besteht, wobei es sich bei dem Mittel zum Einstellen der Osmolalität um mindestens eines handelt, das ausgewählt ist aus der Gruppe bestehend aus Natriumchlorid, Calciumchlorid, Natriumsulfat und Magnesiumchlorid.

11. Zusammensetzung nach Anspruch 10, wobei die Zusammensetzung zu 0,1-30 Gew.-% Taxan, zu 20-99 Gew.-% ein amphiphiles Blockcopolymer mit einem hydrophilen Block und einem hydrophoben Block und zu 0,1-50 Gew.-% ein Mittel zum Einstellen der Osmolalität, bezogen auf das Gesamttrockengewicht der Zusammensetzung, beinhaltet.

12. Zusammensetzung nach Anspruch 10 oder 11, wobei das Taxan Paclitaxel, Docetaxel, 7-Epipaclitaxel, t-Acetylpaclitaxel, 10-Desacetylpaclitaxel, 10-Desacetyl-7-epipaclitaxel, 7-Xylosylpaclitaxel, 10-Desacetyl-7-glutarylpaclitaxel, 7-N,N-Dimethylglycylpaclitaxel, 7-L-Alanylpaclitaxel oder ein Gemisch davon beinhaltet.

13. Zusammensetzung nach einem der Ansprüche 10 bis 12, wobei es sich bei dem hydrophilen Block (A) um Polyethylenglycol oder Monomethoxypolyethylenglycol handelt und es sich bei dem hydrophoben Block (B) um Polymilchsäure oder Copolymer von Polymilchsäure und Glycolsäure handelt.

## Revendications

1. Procédé de préparation d'une composition de micelles polymères contenant des médicaments faiblement solubles, comprenant :

   la dissolution d'un médicament faiblement soluble et d'un copolymère à blocs amphiphile dans un solvant organique pour obtenir une solution, et
   l'ajout d'une solution aqueuse à la solution résultante pour former des micelles polymères,
   où le procédé ne nécessite aucune opération séparée pour éliminer le solvant organique avant l'ajout de la solution aqueuse,
   où le solvant organique est utilisé en une quantité de 0.5 à 15% en poids, sur la base du poids total de compositions, et
   où la solution aqueuse comprend un agent d'ajustement de l'osmolalité qui est au moins un agent choisi dans le groupe constitué du chlorure de sodium, du chlorure de calcium, du sulfate de sodium et du chlorure de magnésium.

2. Procédé selon la revendication 1, où les micelles polymères sont formées par l'ajout de la solution aqueuse à la solution résultante à 0-60°C.

3. Procédé selon la revendication 1 ou 2, où le médicament a une solubilité de 100mg/ml ou moins dans l'eau.

4. Procédé selon l'une quelconque des revendications 1 à 3, où le copolymère à blocs amphiphile est un copolymère dibloc ayant un bloc hydrophile (A) et un bloc hydrophobe (B), où
   le bloc hydrophile (A) est au moins un polymère choisi dans le groupe constitué de polyalkylèneglycol, d'alcool polyvinylique, de polyvinylpyrrolidone, de polyacrylamide et de leurs dérivés, et
   le copolymère hydrophobe (B) est au moins un copolymère choisi dans le groupe constitué de polyester, de polyahnydride, de polyaminoacide, de polyorthoester, de polyphosphazine et de leurs dérivés.

5. Procédé selon la revendication 4, où le copolymère à blocs amphiphile comprend le bloc hydrophile (A) et le bloc hydrophobe (B) dans un rapport pondéral de 3:7 à 8:2 (bloc hydrophile (A): bloc hydrophobe (B)).

6. Procédé selon l'une quelconque des revendications 1 à 5, où le solvant organique est au moins un solvant choisi dans le groupe constitué des alcools, de l'acétone, du tétrahydrofurane, de l'acide acétique, de l'acétonitrile et du dioxane.

7. Procédé selon l'une quelconque des revendications 1 à 6, où la solution aqueuse comprend l'agent d'ajustement de l'osmolalité à une osmolalité de 30-15000mOsm/kg.

8. Procédé selon l'une quelconque des revendications 1 à 7, où un agent de lyophilisation est ajouté à la composition de micelles pour effectuer la lyophilisation, après avoir formé des micelles polymères.

9. Procédé selon l'une quelconque des revendications 1 à 8, où la composition de micelles polymères contenant des médicaments faiblement solubles comprend de 0,1-30% en poids d'un médicament faiblement soluble et de 70-99,9% en poids d'un copolymère à blocs amphiphile contenant un bloc hydrophile et un bloc hydrophobe, sur la base du poids sec total de composition.

10. Composition de micelles polymères sous forme d'une composition lyophilisée contenant un médicament faiblement soluble, où ladite composition consiste en du taxane, un copolymère à blocs amphiphile ayant un bloc hydrophile et un bloc hydrophobe, un agent d'ajustement de l'osmolalité, et un agent de lyophilisation, où l'agent d'ajustement de l'osmolalité est au moins un élément choisi dans le groupe constitué du chlorure de sodium, du chlorure de calcium, du sulfate de sodium et du chlorure de magnésium.

11. Composition selon la revendication 10, où la composition comprend de 0,1-30% en poids de taxane, de 20-99% en poids d'un copolymère à blocs amphiphile ayant un bloc hydrophile et un bloc hydrophobe, et de 0,1-50% en

**EP 2 201 935 B1**

poids d'un agent d'ajustement de l'osmolalité, sur la base du poids sec totale de composition.

12. Composition selon la revendication 10 ou 11, où le taxane comprend le paclitaxel, le docétaxel, le 7-épipaclitaxel, le t-acétyl paclitaxel, le 10-désacétylpaclitaxel, le 10-désacétyl-7-épipaclitaxel, le 7-xylosylpaclitaxel, le 10-désacétyl-7-glutarylpaclitaxel, le 7-N,N-diméthylglycylpaclitaxel, le 7-L-alanylpaclitaxel ou un mélange de ceux-ci.

13. Composition selon l'une quelconque des revendications 10 à 12, où le bloc hydrophile (A) est du polyéthylèneglycol ou du monométhoxypolyéthylèneglycol, et le bloc hydrophobe (B) est de l'acide polylactique ou un copolymère d'acide polylactique et d'acide glycolique.

【Figure 1】

【Figure 2】

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070003625 A1
- WO 0187345 A1
- WO 0245689 A1

- WO 0185216 A1
- US 2004005351 A1
- WO 0333592 A